# EUROPEAN PATENT APPLICATION

(11) **EP 1 483 962 A2**
(43) Date of publication of application: **08.12.2004**
(21) Application number: 04012848.0
(22) Date of filing: 01.06.2004
(51) Int. Cl.: A01K 67/027, C12N 15/11

(54) **Transgenic animal model for Glyt1 function**

(30) Priority: 06.06.2003 EP 03012208
(71) Applicant: F. HOFFMANN-LA ROCHE AG, 4002 Basel (CH)
(72) Inventor: Alberati-Giani, Daniela, 4800 Zofingen (CH); Moehler, Hanns P., 8706 Meilen (CH); Pauly-Evers, Meike, 79111 Freiburg (DE)

(57) **Abstract**

The present invention provides vector constructs and methods for producing non-human transgenic animals comprising within their genome a conditional targeted mutation within the *glyt1* gene which animals could be further used to generate transgenic animals which produce less or no active GLYT1 protein. Moreover, said transgenic animals producing less or no active GLYT1 protein, as well as methods of producing them, are also provided. The invention also relates to the use of these animals as a tool for assessing GLYT1 function and as a model for studying the effects of enhancing synaptic NMDA receptor function and for studying the susceptibility to compounds inducing psychotic behavior, and to methods for evaluating the *in vivo* effects of GLYT1 function and testing GLYT1 inhibitors for effects other than GLYT1-specific effects.

SEQUENCE LISTING

<110>  F. Hoffmann-La Roche AG

<120>  Transgenic animal model for Glyt1 function

<130>  Case 21779

<160>  16

<170>  PatentIn version 3.1

<210>  1
<211>  16057
<212>  DNA
<213>  Artificial

<400>  1
gcggccgcga gctcaattaa ccctcactaa agggagtcga ctcgatccgg atgtctttta      60
gaggaactcc tgtcctttca gggccgtagt tcatctctgg gaggctgttg gtgtacaagg     120
acactgtgtg tgtgctgtgt gtatttcttt actggtgact cctagggtcc ttaggcttcc     180
caatgacaat ctcgggcccc tcaagtctgg cttatggtga atgactcaca ctcataacgg     240
gagagggttc agagaggagt ggcttgggga aaggggacac actaagggac tttcagaggt     300
agttctacat ctgaagaagg aactcctcgt ggagtgtcct cagtgggtcc tggacagttt     360
aacccagcac ttcctgcact caggccgaat acccatcagg gattcatctg caggccaccc     420
tctcattcag tgacaccctt tcctggaacc acctctgttt ccttagccag gcctgtcctc     480
agagtctggc acggtgccca cagctctggt ctcctgccta acctcatagc ccagcattct     540
cctgccatgg ctcaggtcag ccccctctga tctctagtgt cttgcccagg tctggtctag     600
cctagatgca cagccccctc ttccacccag gcctgctcac cgtgtgcccc caggaagaag     660
ccttctctag ggataagtct agccaccgcc tgccatgggc atcgtgctta gcctggcgat     720
gtggtaggcg tcgggtgggg ggtagctgtc cggacacagg cccctttgtt tccggcttga     780
cttggctgct tggtatgagg ctcagcttct cctcaggtcc ttagcctgag aggaatggca     840
agggtttaga ggggttaggg ggctcttggc tcaggctccc tcctcagtgt cccctttcct     900
gctgtaacca gctcctcagg tgggctggta tacctggcat ggagtgctga gggctgggca     960
ctgtctggca gctgtacctc tcctctcttc tgccaggctg gccctcaact cttccggccg    1020
agagggagcc ccagggtatg tggcagggtg atgtggaggg ccaaaggaaa accgctggga    1080
gcgactcaga gggtcagaca cggggcacaa atgcctttac tgatggtagc ttctttcctg    1140
tccccacaga atggtgctgt gcccagcgag gccaccaaga aggaccagaa cctcacacgg    1200
ggcaactggg gcaaccagat cgagtttgta ctgacgagcg tgggctatgc cgtgggcctg    1260

ggcaatgtct ggcgtttccc atacctctgc tatcgcaacg ggggaggtac ccagtgggca 1320
cagggcaggg accacccagt tgggtagagc ccacccaccg gggcttgggc caccatctcc 1380
ccagaacagg tgccactcca ggaagaagct ggcctctggc cctggtgttt acccccatcc 1440
cttaggcata ttttgcctaa ggtgcagacc accctcagat cctgccagcc tgttctctgt 1500
gcctgatctg ctagcctgct ccttgaaagc tagaagtaga ggaaaccact tgaatttcct 1560
gggttctctg tcttaacacc ccatatttca acagtctccc agattgtaca gagatatttg 1620
aggatcccag tgacgtgtgt gtgtgtgtgt gtgtgtgtgt gtgtgtgtgt gtgtgagaga 1680
gagagagaga gagagagaga gagagagaga gagagagaga gagagagaaa tcaattggtt 1740
aaagcttcaa atcctacccc aacatggcag tccggggcag gatttttctg cagagcagac 1800
ttcctgggga gcctctgttc agttcgaaga cagggcaggc agggaatgtg tgtcagcccc 1860
agacagctgc tcagtgcagg caggctcctg tcccagtact agtgaagcac gaaggtttta 1920
ctgtgggttt ggagaagggg acccagggag gggtacctgg agcctaccct gagccctgcc 1980
tcctcctcct cctctgtgcc aggagccttc atgttcccct acttcatcat gctgatcttc 2040
tgcgggattc ctctcttctt catggagctt tccttcggcc agtttgcaag ccagggctgc 2100
ctgggggtct ggcggatcag ccccatgttc aaaggtgagg ttctggtggg gcacgtgcac 2160
accagatgcg cacgtgtcat acacacacac accccagacg cgcacgtgtg atacacgcac 2220
gcggaggcca ctggcttgtc catctgtaca aacacgagca tccccttggt gctgacccca 2280
agcgcaacat caatggccaa ggccatggac acctgctggc tggagcgcat tcatgtttac 2340
tcggccccca cctctgtctc ccgcctcgca gagacacaaa actcctttcc tcccctaact 2400
taccaaggcc tgaaccttcc catctacaac tctccattag gagccgttaa catttagcta 2460
ggcctgcgag gctggctctc actccatggt gctcaccgtg cctttcccac aggcgtgggc 2520
tatggtatga tggtggtgtc cacatacatt ggcatctact acaacgtggt catctgcatc 2580
gccttctact acttcttctc gtccatgacg cacgtgctgc cctgggcgta ctgcaacaac 2640
ccctggaaca cacctgactg tgccggtgtg ctggacgctt ccaacctcac caatggctcc 2700
cggcccgctg ccctgtctgg caacctgtct cacctgttca actacacctt gcaaaggacc 2760
agccccagcg aggagtactg gaggtgaggg gacctggtgg cagcccttag acctcctacc 2820
cccatctggc agccgtgttt tcatcacctg catgtagtgt ctgccgtgca ggggacagag 2880
gcaggttggg gtggcagata tgcagagggc cgtggaaggc ttggaggtga actgggagtc 2940
cttacagcgt aacagtagac gagggctctg gaaatcttaa ggctccagag gcccagtcag 3000
aacaagggga gagagcatgt gatggagggc gtcatgaggg aggtcccaga ggaccagagg 3060
tcattctcaa agctgtggga gagccctatt gagattcaca tgaatggatg gatcctgtgg 3120
gctttgaggt ggccttgaag ggcactacgt tcacagagaa actaaccaga gttaagcttc 3180
ctcttcatac tgtagaacaa aaggagccat gagcattcac taggccaggg atgccaggac 3240
agccaggccc gttctgttca gaggtgacaa agagactagt gtctctgggc cctctgaagg 3300
aaggcaacta ctggaggtgt ctgtctggac caccttatcc caactggcag atggaaggcg 3360
cagtggggta ttcttcctac atcttactgg agagtcctgg ggcaaaggac ctttgcatgt 3420
gggaggtaga cctcatctat ttattctgta ggtgtctgct ctaaaggctc tgtgtgccca 3480
tggcggtgta aaatggtcca gagcctctgt cccgagagag atcacatagt ctatagagaa 3540
acagatagtt cagtgcatta ggagcaggtg ccctgcctgg cacagaagta tggaagtggc 3600

actaagaagg tgctggagac ttctgagaga tggggacaag gggagctggt gataactgcc 3660
cagacatggg aggaaagagt taggaggaat tcacaagtag gtgggcacag aggtccacaa 3720
ggaggcaaac tgacgccaag gttgggaaca aatgggacac tgagaaaaga gacaaggatg 3780
agcaccatca tatggtgcca gcttctacgg cagccaaaag gcttgaccat ttccctgctg 3840
tgtgatggag gccaaaggtg aggtgtgcat ggctctgggc gcacagaagt tgtagcaggt 3900
cctctaaatc cagtgctgaa agtgcaaatc ccacacagct gtggggaggg gccactccca 3960
gagaggagat gaggaagaaa gggagagaga aatctcaccc aatcagatga actgtccacc 4020
aggggtgtga tgcgcacagg atggcccaga cggaaaggca gaatatgaac ttccctggag 4080
ctccccaaaa gcacaaaccc tgcatgccaa ccttagccca ggcagatgct ccagtgttgc 4140
ctgcctcagg ggcaaagctc ctgagactgg agccagtgac aatcagtggg gaatagctct 4200
cagagaatct gcctagcggg gcatactgag gggacagctg ggccctgctc cgcacctggg 4260
gctgtgccag gcctacctac ctagctcagt tcctaccccc caatggctcc ctgctgccac 4320
ctcctggaga gcctggaaag atatttctcc cctgctatac tggtgattga ccactgcccc 4380
atgcccagat ccatgcaata acttcgtata gcatacatta tacgaagtta tgaattctgc 4440
atgccaggca aatgcctcat ccctgagctc tgtccctagc cgccttctta cattttcatt 4500
ttgaggttgg tcttgaactt gtgctcctcc tgcccaagct ctagagctcg ctgtcagagc 4560
tggagcagat ggttaagggt atacctaaca gcttcctgga gggtgcactt ttgataggat 4620
gagtgggcat tcctactgtg gaatgcacgt gaaaggacct gagctgagcc caagcagaga 4680
cttgggagtc atcctgtccc aggaggaaag actagctcaa acttgattta atctctcaga 4740
gcccttgagg gggtgcccag ggcagactgc agggcagagg cctggggtcc tcaaagcagc 4800
ttccgttctc ccacatgcag gcttctgggg tcttgcagca tggggcatgg atcatgagaa 4860
ttggttgctg ggcggcagag gctggccctc agtgacggtc acctccccat ccccaggctg 4920
tatgtgctga agctgtcaga cgacattgga aactttgggg aagtgcggct tcccctccta 4980
ggctgcctcg gcgtctcctg ggtggttgtc ttcctctgtc tcatccgagg agtcaagtct 5040
tcagggaaag caagtagcct ccccaggcag ggtttgttcc caccctgcct ctcctatctg 5100
ggttgccggg gttagggagg taatgcgggg gttggagagg gctctagggg aatccatggc 5160
ccctgtcttg tcccaggtgg tgtacttcac ggccacattc ccctatgtgg tgctgaccat 5220
tctgtttgtt cgtggagtga ccctggaagg agccttcacg ggtatcatgt actacctgac 5280
cccacaatgg gacaagtacc tggaggccaa ggtgggatgt ggagggttga tccccaaagg 5340
gaccaaggcg ggtgcatcta acatctgcct gcagtggctc actggccatc ctttctgcag 5400
gtctgggggg atgcagcctc tcagatcttc tattccctgg gctgtgcatg gggtggcctc 5460
atcaccatgg cgtcctacaa caaatttcac aacaactgct accggtgagt gagcaccccc 5520
cgccccctgc tccctcagcc tggaggcttc ccgtcttccc atcctctccc cagcctgacc 5580
ctggatccac agggccattg ggctccagtg cagctggctg cctcccagtg acctcctgca 5640
cccatgacct tgaagtcttg aggatcttgt atctccagtg tgaaagtaga aggcaaagca 5700
ccagccaaag agggggaatg gggtagatga agggtcagga accctggact cgactttaaa 5760
aggcccagat agacaaggag ttagggttac ctttcttttt agaatatcta atccctgtgt 5820
cttcccctgg cctaaattga ggaaagggaa gcaagctggg tctcctaaac tgtcccgttt 5880

tccctccctc agggacagcg tcattatcag tatcaccaac tgtgctacca gcgtctacgc 5940
cggcttcgtc atcttctcca tcctgggctt catggccaat cacctgggtg tggatgtgtc 6000
tcgggtggca gaccatgggc ctgggctagc ttttgtcgct taccctgagg ctctcacgct 6060
gcttcccatc tctccactct ggtccttatt gtttttcttc atgctcatct tgctggggct 6120
gggtactcag gtatggggtg tgtgtatgtg tgtgtgtgtg tgtgtgtgtg tgtgtgtgtg 6180
tgtgttttga gggagtggtc tgggacaaaa cgcgggccct tattctgata gccgtggctg 6240
tagttctgcc tcctagagac cctggtcact gccattgtgg atgaggtagg gaatgagtgg 6300
attctgcaga agaagaccta cgtgaccttg ggcgtcgctg tggctggctt cttgctgggc 6360
atccccctta ccagccaggt aagaatggag atgtgggggt gaggcggggc tgcaatcgcc 6420
aggcacttgg gaatggatga gtagcctagt ctgtggaacc ctggcttacc tgcccactgg 6480
cccacaggca ggcatctact ggctgctgct gatggacaac tatgcagcca gcttctcctt 6540
ggttgtcatc tcctgtatca tgtgtgtatc catcatgtac atctatggtg agtgctaccc 6600
ttccgtggac tcgagtgtcc gtctcggact ggcccactct gttctcctga gctgctgaca 6660
atatctccca cccctgtctc tccagggcac cgtaactact tccaggacat ccagatgatg 6720
ctaggatttc cccgcctctc ttcttccag atctgttggc gctttgtttc tccggctatc 6780
atctttgtaa gttcctgggg ggggggggct cccaggtagc tctagctttg ggcatctaag 6840
aaggccagat agtcagaggg taccataact tcgtatagca tacattatac gaagttattt 6900
cgaagttcct attctctaga aagtatagga acttcaagct aattctaccg ggtaggggag 6960
gcgcttttcc caaggcagtc tggagcatgc gctttagcag ccccgctggg cacttggcgc 7020
tacacaagtg gcctctggcc tcgcacacat tccacatcca ccggtaggcg ccaaccggct 7080
ccgttctttg gtggcccctt cgcgccacct tctactcctc ccctagtcag gaagttcccc 7140
cccgccccgc agctcgcgtc gtgcaggacg tgacaaatgg aagtagcacg tctcactagt 7200
ctcgtgcaga tggacagcac cgctgagcaa tggaagcggg taggcctttg gggcagcggc 7260
caatagcagc tttgctcctt cgctttctgg gctcagaggc tgggaagggg tgggtccggg 7320
ggcgggctca ggggcgggct caggggcggg gcgggcgcga aggtcctccg gagcccggca 7380
ttctgcacgc ttcaaaagcg cacgtctgcc gcgctgttct cctcttcctc atctccgggc 7440
ctttcgacct gcagccaata tgggatcggc cattgaacaa gatggattgc acgcaggttc 7500
tccggccgct tgggtggaga ggctattcgg ctatgactgg gcacaacaga caatcggctg 7560
ctctgatgcc gccgtgttcc ggctgtcagc gcaggggcgc ccggttcttt ttgtcaagac 7620
cgacctgtcc ggtgccctga atgaactgca ggacgaggca gcgcggctat cgtggctggc 7680
cacgacgggc gttccttgcg cagctgtgct cgacgttgtc actgaagcgg gaagggactg 7740
gctgctattg ggcgaagtgc cggggcagga tctcctgtca tctcaccttg ctcctgccga 7800
gaaagtatcc atcatggctg atgcaatgcg gcggctgcat acgcttgatc cggctacctg 7860
cccattcgac caccaagcga aacatcgcat cgagcgagca cgtactcgga tggaagccgg 7920
tcttgtcgat caggatgatc tggacgaaga gcatcagggg ctcgcgccag ccgaactgtt 7980
cgccaggctc aaggcgcgca tgcccgacgg cgaggatctc gtcgtgaccc atggcgatgc 8040
ctgcttgccg aatatcatgg tggaaaatgg ccgcttttct ggattcatcg actgtggccg 8100
gctgggtgtg gcggaccgct atcaggacat agcgttggct acccgtgata ttgctgaaga 8160
gcttggcggc gaatgggctg accgcttcct cgtgctttac ggtatcgccg ctcccgattc 8220

gcagcgcatc gccttctatc gccttcttga cgagttcttc tgaggggatc aattctctag 8280
agctcgctga tcagcctcga ctgtgccttc tagttgccag ccatctgttg tttgcccctc 8340
ccccgtgcct tccttgaccc tggaaggtgc cactcccact gtcctttcct aataaaatga 8400
ggaaattgca tcgcattgtc tgagtaggtg tcattctatt ctggggggtg gggtggggca 8460
ggacagcaag ggggaggatt gggaagacaa tagcaggcat gctggggatg cggtgggctc 8520
tatggcttct gaggcggaaa gaaccagctg gggctcgatc gagatccgaa gttcctattc 8580
tctagaaagt ataggaactt ccctgcacac tcagagaggt gccaggtcct ggccatgggt 8640
taggaagtgg atcttgagcc ctctgtttga ctgaagccag taacatatgc tcagagctgc 8700
aatgtcaggg acacagcctc actccatgcc attacttgcc cagttgcctc caaggggcct 8760
cgggagtcat tgggatcagt gtggcttctg gtctacttta ctgttcacac cttagcccca 8820
tggaaggctg ccctaagcag ggagacacat aggaatgtgg cagcatttct ccacagcccc 8880
aggacctgag ggtacagggc taggagtcag gttgactctg tgatgggact aagccctcaa 8940
aggctgggaa gttgaatggc cttgaaggga gcacccccat ttctttgaac atcaagcatc 9000
aggggaatgt atcagagcac actgcactgc tgataggatt ccctgcaagg ccggtccttc 9060
ctccaccctg ttcacatgtt aacttgagca acactctctg gatcatcaat tctccccact 9120
ctaaggcaca gggtatgtta aataagccca ccccacactt aataaggggc aagaaacctg 9180
ctgcaggcct gtgggctaac ccaggcgcat tttgctgctt ctgtttgaag tgggaggtgg 9240
ggctaatggc ctaatcatca ctatatgaga aaagagggtc agagggctca ctcaatggca 9300
gggcagtgtc cagagctacc tgtgtcttgc tcagttctcc gaattgtgga aagcgctaga 9360
ccagacttcc acactggctc atatttctga aaagaggtcc ctggaggacc gaggcctcca 9420
ccgcatgggg tgtgggatgt tcctgggcct ggcacagaca acagttctgg ccacaggccc 9480
agcattgaaa caggaggagc ggaagtctcc tttcaaggct gagagggcag agagggcggg 9540
aaccactgcc cagcgccatg ccttctgggg gcgtggcatt ggcccagaac caggacgctc 9600
tctgagggct ggggagctgc actttggtac ctgagtttac ccaccaaggg ccttccctgg 9660
gttctgtgga ttgtctaggc agagagattg aatcaggcag tgtccaaaag ctgggttgga 9720
gttacattaa gatgaggaat tcaggccggg cagacatatt tctcttcgga gctttatgtg 9780
cctgtgacag ctcttcttga caaacccctt ccttccttca taactgcagt gaaaaaattc 9840
ctcacataca tcaggctttg ttaagccagg tgcggtggcg ctcgcattta atctcagcct 9900
tggggaggca agccattgtc tgaatttgag gccagcttgg tctacatagg gagttttagg 9960
ttagctaggg tgacacaagt gagaccctgt cctgaaaaaa aaaaaaaaaa aaaacaaaag 10020
caaacacaca cacacacact cactccacta ggttagtaaa gatgtcacac acatatggtg 10080
ccctaggttc agtgtgggga aggtcagagg aacttaagct ccgggatgcc cctaaatgct 10140
gacaggcatg gcgagtcctt tctacaggtc ctctggtgtg gcctgtgagc cacctggcca 10200
agtctcctgt ttcttttaga tctttagctc tcctctttcc agagaggtat gtgtctgcta 10260
ctggacacag catctgtagc ccctgggcac cctccaagct gtcacaacaa gctggcttgt 10320
tatcacagcc acagtgaggc aagactttac gtagcttgct cctcggcccc cttcctgatc 10380
tgagcatctc ccggtacaaa tcagctcaaa tagataaatg agattgtgca agacctcaga 10440
cccacagtca gtgcggtaac ctcaagtgtc aggccatgca gtacaggaag ccgagtgatt 10500

ttgagttaaa cagatgggag gtgccatgga gaagcctttt aggaggcaga aaatccagaa 10560
aggcttcctg aaagaggtgg cactggctca gggcttgaca ggaatgccga ggatgggtct 10620
gtagaatgaa cagaggcggc taagacggtc tggatgtggt gaacgcctag ggcagggaga 10680
tgggaccagc aaatcacagg ccatcaggca gagctgaagc tacggctagg aaatcatcct 10740
tagctctact gcctagagag gacggctgct tttcaaggtg gcagccgctt acttgttctc 10800
tgccagttca ggactctggc aaaaaccgtg tcttcatgcc cagcccctcc ctggggtcca 10860
acttctttca cgaaggtgtt gacagttgcc tatgccccga gccacaccac cttttgctga 10920
cctaggagga gggccagcct ggggtcccag gaggggcgct actccgcctc acatcatcct 10980
ctctaccctc tctagttcat tctcatcttc acggtgatcc agtaccggcc aatcacctac 11040
aaccactacc agtacccagg ctgggctgtg cgcatcggct tcctcatggc tttgtcgtct 11100
gtcatctgca tcccactgta cgcgctgttc cagctctgcc gcacagatgg ggacacactt 11160
cttcaggtga ggagtgaaga tggactgaac ggggacgctg gacgggacgg gcaggcgaag 11220
tggaaacact tcgagcataa gggttctctt ctgacacgct tttcactgcg ccccctgcct 11280
gcagcgtttg aaaaatgcca caaagccaag cagagactgg ggccccgctc tcctggagca 11340
ccggactggg cgctacgctc ccactacaac cccctctccc gaagacgggt ttgaggtcca 11400
gccactgcac ccggacaagg cccagattcc catcgtgggc agtaacggct ccagccgctt 11460
ccaggactcc cggatatgag cgcaattgtt gcaaggggag aaaccccacc caacccttgc 11520
tcccaccacg gagactggga ggcggacagg tggaggcctg ccccatcaca ccctgtctgg 11580
gatggctgct gtcaccttga ccaccactgc tcatgtcccc agtgtttaca catcctttgg 11640
atgccaagac agcagctggg ggtgggaaag cgatggggga attgggggga gcgggcatgg 11700
ggaagcagtg tccaaagcac tttggaggag gcctgccagg cttcctatgg cctctgatac 11760
cctcatgctc tgccctgagc tgtttcacta tgggataaga ggttctgtgt ccacatccca 11820
ccggttcccc tcagcctcgt gaccagtgtt cctgacccca gaacagaccc tagcctctgc 11880
ccaggaaaat ctgtatcttc ctcctccaga caaggtgagg gcccccgtcg agaccgaaca 11940
actccgccgc gcgcgctccg tgtaggccgg tgcgggcggc cccgtagcgc aagggagggc 12000
gggaaaggaa gggcgggaca caagggcgaa tctataaagg gcgtcactca gccagttctc 12060
tcctcagaag cgccgagagc gcgaccggga cggttggaga agaaggtggc tcccggaagg 12120
gggagagaca aactgccgta acctctgccg ttcaggatct gcgacctgca ggtcctcgcc 12180
atggaccctg atgatgttgt tgattcttct aaatcttttg tgatggaaaa cttttcttcg 12240
taccacggga ctaaacctgg ttatgtagat tccattcaaa aaggtataca aaagccaaaa 12300
tctggtacac aaggaaatta tgacgatgat tggaaagggt tttatagtac cgacaataaa 12360
tacgacgctg cgggatactc tgtagataat gaaaacccgc tctctggaaa agctggaggc 12420
gtggtcaaag tgacgtatcc aggactgacg aaggttctcg cactaaaagt ggataatgcc 12480
gaaactatta agaaagagtt aggtttaagt ctcactgaac cgttgatgga gcaagtcgga 12540
acggaagagt ttatcaaaag gttcggtgat ggtgcttcgc gtgtagtgct cagccttccc 12600
ttcgctgagg ggagttctag cgttgaatat attaataact gggaacaggc gaaagcgtta 12660
agcgtagaac ttgagattaa ttttgaaacc cgtggaaaac gtggccaaga tgcgatgtat 12720
gagtatatgg ctcaagcctg tgcaggaaat cgtgtcaggc gatctctttg tgaaggaaac 12780
cttacttctg tggtgtgaca taattggaca aactacctac agagatttaa agctctaagg 12840

taaatataaa atttttaagt gtataatgtg ttaaactact gattcctaat tgtttgtgta 12900
ttttagattc caacctatgg aactgatgaa tgggagcagt ggtggaatgc agatccacta 12960
ggatctaact tgtttattgc agcttataat ggttacaaat aaagcaatag catcacaaat 13020
ttcacaaata aagcattttt ttcactgcat tctagttgtg gtttgtccaa actcatcaat 13080
gtatcttatc atgtctggat cgtagttcta gagcggactc gacgaatggg ctgcaggaat 13140
tcgatatcaa gcttatcgat accgtcgacc tcgagggggg gcccggtacc cagcttttgt 13200
tccctttagt gagggttaat tgcgcgcttg gcgtaatcat ggtcatagct gtttcctgtg 13260
tgaaattgtt atccgctcac aattccacac aacatacgag ccggaagcat aaagtgtaaa 13320
gcctggggtg cctaatgagt gagctaactc acattaattg cgttgcgctc actgcccgct 13380
ttccagtcgg gaaacctgtc gtgccagctg cattaatgaa tcggccaacg cgcggggaga 13440
ggcggtttgc gtattgggcg ctcttccgct tcctcgctca ctgactcgct gcgctcggtc 13500
gttcggctgc ggcgagcggt atcagctcac tcaaaggcgg taatacggtt atccacagaa 13560
tcaggggata acgcaggaaa gaacatgtga gcaaaaggcc agcaaaaggc caggaaccgt 13620
aaaaaggccg cgttgctggc gtttttccat aggctccgcc cccctgacga gcatcacaaa 13680
aatcgacgct caagtcagag gtggcgaaac ccgacaggac tataaagata ccaggcgttt 13740
ccccctggaa gctccctcgt gcgctctcct gttccgaccc tgccgcttac cggatacctg 13800
tccgcctttc tcccttcggg aagcgtggcg ctttctcata gctcacgctg taggtatctc 13860
agttcggtgt aggtcgttcg ctccaagctg ggctgtgtgc acgaaccccc cgttcagccc 13920
gaccgctgcg ccttatccgg taactatcgt cttgagtcca acccggtaag acacgactta 13980
tcgccactgg cagcagccac tggtaacagg attagcagag cgaggtatgt aggcggtgct 14040
acagagttct tgaagtggtg gcctaactac ggctacacta gaaggacagt atttggtatc 14100
tgcgctctgc tgaagccagt taccttcgga aaaagagttg gtagctcttg atccggcaaa 14160
caaaccaccg ctggtagcgg tggttttttt gtttgcaagc agcagattac gcgcagaaaa 14220
aaaggatctc aagaagatcc tttgatcttt tctacggggt ctgacgctca gtggaacgaa 14280
aactcacgtt aagggatttt ggtcatgaga ttatcaaaaa ggatcttcac ctagatcctt 14340
ttaaattaaa aatgaagttt taaatcaatc taaagtatat atgagtaaac ttggtctgac 14400
agttaccaat gcttaatcag tgaggcacct atctcagcga tctgtctatt tcgttcatcc 14460
atagttgcct gactccccgt cgtgtagata actacgatac gggagggctt accatctggc 14520
cccagtgctg caatgatacc gcgagaccca cgctcaccgg ctccagattt atcagcaata 14580
aaccagccag ccggaagggc cgagcgcaga agtggtcctg caactttatc cgcctccatc 14640
cagtctatta attgttgccg ggaagctaga gtaagtagtt cgccagttaa tagtttgcgc 14700
aacgttgttg ccattgctac aggcatcgtg gtgtcacgct cgtcgtttgg tatggcttca 14760
ttcagctccg gttcccaacg atcaaggcga gttacatgat cccccatgtt gtgcaaaaaa 14820
gcggttagct ccttcggtcc tccgatcgtt gtcagaagta agttggccgc agtgttatca 14880
ctcatggtta tggcagcact gcataattct cttactgtca tgccatccgt aagatgcttt 14940
tctgtgactg gtgagtactc aaccaagtca ttctgagaat agtgtatgcg gcgaccgagt 15000
tgctcttgcc cggcgtcaat acgggataat accgcgccac atagcagaac tttaaaagtg 15060
ctcatcattg gaaaacgttc ttcggggcga aaactctcaa ggatcttacc gctgttgaga 15120

tccagttcga tgtaacccac tcgtgcaccc aactgatctt cagcatcttt tactttcacc 15180
agcgtttctg ggtgagcaaa aacaggaagg caaaatgccg caaaaaaggg aataagggcg 15240
acacggaaat gttgaatact catactcttc ctttttcaat attattgaag catttatcag 15300
ggttattgtc tcatgagcgg atacatattt gaatgtattt agaaaaataa acaaataggg 15360
gttccgcgca catttccccg aaaagtgcca cctaaattgt aagcgttaat attttgttaa 15420
aattcgcgtt aaatttttgt taaatcagct cattttttaa ccaataggcc gaaatcggca 15480
aaatccctta taaatcaaaa gaatagaccg agatagggtt gagtgttgtt ccagtttgga 15540
acaagagtcc actattaaag aacgtggact ccaacgtcaa agggcgaaaa accgtctatc 15600
agggcgatgg cccactacgt gaaccatcac cctaatcaag ttttttgggg tcgaggtgcc 15660
gtaaagcact aaatcggaac cctaaaggga gcccccgatt tagagcttga cggggaaagc 15720
cggcgaacgt ggcgagaaag gaagggaaga aagcgaaagg agcgggcgct agggcgctgg 15780
caagtgtagc ggtcacgctg cgcgtaacca ccacacccgc cgcgcttaat gcgccgctac 15840
agggcgcgtc ccattcgcca ttcaggctgc gcaactgttg ggaagggcga tcggtgcggg 15900
cctcttcgct attacgccag ctggcgaaag ggggatgtgc tgcaaggcga ttaagttggg 15960
taacgccagg gttttcccag tcacgacgtt gtaaaacgac ggccagtgag cgcgcgtaat 16020
acgactcact atagggcgaa ttggagctct agagctc 16057

<210> 2

<211> 26

<212> DNA

<213> Mus musculus


<400> 2
ccctttcctg gaaccacctc tgtttc 26

<210> 3

<211> 26

<212> DNA

<213> Mus musculus


<400> 3
tgcgatagca gaggtatggg aaacgc 26

<210> 4

<211> 26

<212> DNA

<213> Mus musculus


<400> 4
catgctgatc ttctgcggga ttcctc 26

<210> 5

<211>  26

<212>  DNA

<213>  Mus musculus


<400>  5
ggctggtcct ttgcaaggtg tagttg                                          26


<210>  6

<211>  26

<212>  DNA

<213>  Mus musculus


<400>  6
gctgtatgtg ctgaagctgt cagacg                                          26


<210>  7

<211>  26

<212>  DNA

<213>  Mus musculus


<400>  7
gatgatagcc ggagaaacaa agcgcc                                          26


<210>  8

<211>  26

<212>  DNA

<213>  Mus musculus


<400>  8
ctcatcttca cggtgatcca gtaccg                                          26


<210>  9

<211>  26

<212>  DNA

<213>  Mus musculus


<400>  9
aaaccctagt tctgcacaca ctgggc                                          26


<210>  10

<211>  21

<212>  DNA

<213>  Artificial

<400> 10
ggtctgagag tatgtcagag c 21

<210> 11

<211> 23

<212> DNA

<213> Artificial

<400> 11
tgctatacga agttattgca tgg 23

<210> 12

<211> 22

<212> DNA

<213> Artificial

<400> 12
ttccaggaca tccagatgat gc 22

<210> 13

<211> 20

<212> DNA

<213> Artificial

<400> 13
agacacactg gacccaaggc 20

<210> 14

<211> 22

<212> DNA

<213> Artificial

<400> 14
cattgaatgg ggttagcacg tc 22

<210> 15

<211> 20

<212> DNA

<213> Artificial

<400> 15
acatcgcatc gagcgagcac 20

<210>  16

<211>  20

<212>  DNA

<213>  Artificial

<400>  16
tggcacctct ctgagtgtgc                                                    20

## Description

Glycine is the major inhibitory neurotransmitter in the spinal cord and brainstem and is also a co-agonist at the NMDA receptor. The extracellular concentration of glycine is regulated by at least two Na⁺/Cl⁻-dependent glycine transporters (GLYT1 and GLYT2) which play an important role in the termination of post-synaptic glycinergic actions and maintenance of low extracellular glycine concentration by re-uptake of glycine into presynaptic nerve terminals and surrounding fine glial processes. GLYT2 is expressed at high levels in the rodent spinal cord, brainstem and cerebellum where its expression correlates very well with the presence of strychnine-sensitive glycine receptors (Zafra, F., et al., J Neurosci, 1995. 15(5 Pt 2): p. 3952-69; Luque, J.M., N. Nelson, and J.G. Richards, Neuroscience, 1995. 64(2): p. 525-35; Jursky, F. and N. Nelson, J Neurochem, 1995. 64(3): p. 1026-33). Immunohistochemical analysis suggests a predominantly pre-synaptic localization in presumptive glycinergic neurons (Zafra, F., et al., J Neurosci, 1995. 15(5 Pt 2): p. 3952-69 and Spike, R.C., et al., Neuroscience, 1997. 77(2): p. 543-51), strongly suggesting a role in the termination of glycinergic inhibitory synaptic transmission. Human GLYT2 has been cloned and appears to exhibit a similar expression pattern (Morrow, J.A., et al., FEBS Lett, 1998. 439(3): p. 334-40). GLYT2 has some degree of heterogeneity. Indeed two GLYT2 isoforms (2a and 2b) have been identified in rodent brains.

GLYT1 can be distinguished pharmacologically from GLYT2 by its sensitivity to blockade by sarcosine, and N-methylated derivative of glycine (Liu, Q.R., et al., J Biol Chem, 1993. 268(30): p. 22802-8 and Kim, K.M., et al., Mol Pharmacol, 1994. 45(4): p. 608-17). The human *glyt1* gene has been cloned and encodes three isoforms GLYT1a, 1b, and 1c (Kim, K.M., et al., Mol Pharmacol, 1994. 45(4): p. 608-17) whereas only two rat isoforms, GLYT1a and 1b, have been identified (Guastella, J., et al., Proc Natl Acad Sci U S A, 1992. 89(15): p. 7189-93; Smith, K.E., et al., Neuron, 1992. 8(5): p. 927-35 and Borowsky, B., E. Mezey, and B.J. Hoffman, Neuron, 1993. 10(5): p. 851-63). GLYT1 appears to be expressed in both glia cells and neurons in the rat CNS (Zafra, F., et al., J Neurosci, 1995. 15(5 Pt 2): p. 3952-691; Smith, K.E., et al., Neuron, 1992. 8(5): p. 927-35; Smith, K.E., et al., Neuron, 1992. 8(5): p. 927-35; Borowsky, B., E. Mezey, and B.J. Hoffman, Neuron, 1993. 10(5): p. 851-63 and Zafra, F., et al., Eur J Neurosci, 1995. 7(6): p. 1342-52) with GLYT1a apparently expressed in the gray matter as well as in some peripheral tissues whilst GLYT1b is expressed only in the white matter of the CNS KM/22.3.2004 (Borowsky, B., E. Mezey, and B.J. Hoffman, Neuron, 1993. 10(5): p. 851-6310). In humans, a probe common to all GLYT1 isoforms revealed expression in several peripheral tissues, most notably the kidney, whereas GLYT1c seems to be brain specific (Kim, K.M., et al., Mol Pharmacol, 1994. 45(4): p. 608-177). The GLYT1 isoforms differ only in their amino termini and 5' non-coding regions (Kim, K.M., et al., Mol Pharmacol, 1994. 45(4): p. 608-177 and Borowsky, B., E. Mezey, and B.J. Hoffman, Neuron, 1993. 10(5): p. 851-63). GLYT1a and GLYT1b originate from transcription directed from alternate promoters whereas human GLYT1c is a splice variant of the GYT1b transcript (Kim, K.M., et al., Mol Pharmacol, 1994. 45(4): p. 608-17; Adams, R.H., et al., Neurosci, 1995. 15(3 Pt 2): p. 2524-327 and Borowsky, B. and B.J. Hoffman, J Biol Chem, 1998. 273(44): p. 29077-85). The peripheral expression of GLYT1 and the differential CNS expression patterns of the isoforms are somewhat controversial with major discrepancies evident between the published studies. GLYT1 is expressed together with GLYT2 in the spinal cord, brainstem and diencephalon. Interestingly GLYT1 is also expressed in forebrain areas such as the cortex, hippocampus and olfactory bulb where no inhibitory glycinergic neurons have been found (Zafra, F., et al., J Neurosci, 1995. 15(5 Pt 2): p. 3952-691; Guastella, J., et al., Proc Natl Acad Sci U S A, 1992. 89(15): p. 7189-93; Smith, K.E., et al., Neuron, 1992. 8(5): p. 927-35; Borowsky, B., E. Mezey, and B.J. Hoffman, Neuron, 1993. 10(5): p. 851-63 and Zafra, F., et al., Eur J Neurosci, 1995. 7(6): p. 1342-52) thus, suggesting additional roles for GLYT1, which might include regulation of NMDA receptor-mediated neurotransmission (Smith, K.E., et al., Neuron, 1992. 8(5): p. 927-359).

Binding of both glutamate and glycine is necessary for NMDA receptor activation. Whilst glutamate is released in an activity-dependent manner from pre-synaptic terminals, glycine is apparently present at a more constant level, indicating a more modulatory function. Measurements of glycine concentration in the extracellular and cerebrospinal fluids suggest that it is present at low micromolar levels (Westergren, I., et al., J Neurochem, 1994. 62(1): p. 159-6514). However glycine transporters might reduce the glycine concentration markedly in the local microenvironment of NMDA receptors. Indeed, expression of GLYT1b in *Xenopus* oocytes has been shown to reduce the glycine concentration at co-expressed NMDA receptors (Supplisson, S. and C. Bergman, J Neurosci, 1997. 17(12): p. 4580-9015 and Chen, L.; J Neurophysiol, 2003. 89(2): p. 691-703). Additionally, recent studies have suggested that glycine uptake mechanisms can regulate synaptic NMDA receptor activity (Berger, A.J., S. Dieudonne, and P. Ascher, J Neurophysiol, 1998. 80(6): p. 3336-40 and Bergeron, R., et al., Proc Natl Acad Sci U S A, 1998. 95(26): p. 15730-4). NMDA receptor glycine affinity is influenced by the identity of the receptor NR2 subunit and in recombinant systems, receptors containing NR2A exhibit a markedly reduced affinity for glycine relative to those containing NR2B, C or D (Ikeda, K., et al., FEBS Lett, 1992. 313(1): p. 34-8; Kutsuwada, T., et al., Nature, 1992. 358(6381): p. 36-41 and Priestley, T., et al., Mol Pharmacol, 1995. 48(5): p. 841-8). It has been demonstrated that a population of NMDA receptors with a markedly lower affinity for glycine appears during maturation, paralleling the developmental increase in expression of NR2A (Kew, J.N., et al., J Neurosci, 1998. 18(6): p. 1935-4321) suggesting the existence of a population of NMDA receptors not saturated by glycine under normal physiological conditions. Glutamate neurotransmission, in particular NMDA receptor activity, plays a critical role in synaptic plasticity, learning and memory, such as the NMDA receptors appears to serve as a graded switch for gating the threshold of synaptic plasticity and memory formation (Hebb, D., 1949, New York: Wiley and Bliss, T.V. and G.L. Collingridge, Nature, 1993. 361(6407): p. 31-9). Transgenic mice overexpressing the NMDA NR2B subunit exhibit enhanced synaptic plasticity and superior ability in learning and memory (Tang, Y.P., et al., Nature, 1999. 401(6748): p. 63-9).

Facilitation of NMDA receptor activity could be achieved by selectively inhibiting GLYT 1 or by reducing or eliminating its function through genetic intervention. Such approaches should lead to an elevation of the local glycine concentration whilst leaving the regulation of inhibitory glycinergic function by GLYT2 unaffected.

Such a facilitation of synaptic NMDA receptor function would be predicted to produce a cognitive enhancing effect. In support of this strategy, the NMDA receptor glycine site partial agonist D-cycloserine has been shown to enhance acquisition of the water maze task in aged rats (Aura, J., M. Riekkinen, and P. Riekkinen, Jr., Eur J Pharmacol, 1998. 342(1): p. 15-20) and to partially alleviate the deficit in this task induced by medial septal lesion (Riekkinen, P., Jr., S. Ikonen, and M. Riekkinen, Neuroreport, 1998. 9(7): p. 1633-7). Mutant mice with a mild reduction in NMDA receptor glycine affinity also exhibit a deficit in hippocampal LTP and in spatial learning in the Morris water maze (Kew, J.N., et al., J Neurosci, 2000. 20(11): p. 4037-49).

NMDA receptor hypofunction has been implicated in the pathophysiology of schizophrenia (Olney, J.W. and N.B. Farber, Arch Gen Psychiatry, 1995. 52(12): p. 998-1007 and Hirsch, S.R., et al., Pharmacol Biochem Behav, 1997. 56(4): p. 797-802). Non-competitive NMDA receptor antagonists such as PCP and ketamine can induce schizophrenia-like psychosis (Allen, R.M. and S.J. Young, Am J Psychiatry, 1978. 135(9): p. 1081-4; Javitt, D.C. and S.R. Zukin, Am J Psychiatry, 1991. 148(10): p. 1301-8 and Krystal, J.H., et al., Arch Gen Psychiatry, 1994. 51(3): p. 199-214). PCP-induced hyperactivity in rodents is inhibited by glycine and its derivatives (Toth, E. and A. Lajtha, Neurochem Res, 1986. 11(3): p. 393-400 and Toth, E., et al., Comm Psychol Psychiat Behav, 1986. 11: p. 1-9) and interestingly also by glycyldodecylamide (Javitt, D.C., et al., Neuropsychopharmacology, 1997. 17(3): p. 202-4) which inhibits forebrain glycine uptake (Javitt, D.C. and M. Frusciante, Psychopharmacology (Berl), 1997. 129(1): p. 96-8).

Finally, glycine and D-cycloserine have also been shown to significantly reduce the negative and cognitive symptoms in neuroleptic partially responsive schizophrenic patients (Waziri, R., Biol Psychiatry, 1988. 23(2): p. 210-1; Javitt, D.C., et al., Am J Psychiatry, 1994. 151(8): p. 1234-6 and Goff, D.C., et al., Am J Psychiatry, 1995. 152(8): p. 1213-5).

The GLYT1 knockout mouse provides a valuable tool to assess the physiological function of GLYT 1 and provides also a pure GLYT2 expression system. These mice should exhibit elevated levels of glycine in the forebrain and they might be useful in addressing the question of whether active regulation of NMDA receptor glycine site occupancy is important for physiological NMDA receptor function.

The present invention provides vector constructs and methods for producing non-human transgenic animals comprising within their genome a conditional targeted mutation within the *glyt1* gene. These transgenic animals could be further used to generate transgenic animals, which produce less or no active GLYT1 protein. Moreover, said transgenic animals producing less or no active GLYT1 protein, as well as methods of producing them, are also provided. The invention also relates to the use of these animals as a tool for assessing GLYT1 function and as a model for studying the effects of enhancing synaptic NMDA receptor function and studying the susceptibility to compounds inducing psychotic behavior, and to methods for evaluating the *in vivo* effects of GLYT1 function and testing GLYT1 inhibitors for effects other than GLYT1-specific effects.

The present invention therefore provides a vector construct comprising the whole or part of the genomic sequence of the *glyt1* gene, wherein the genomic sequence comprises a positive selection marker and optionally a negative selection marker and wherein at least part of an exon of *glyt1* gene is framed by recognition sites for a recombinase.

The genomic sequence of the *glyt1* gene of the mouse comprises exons 1a, 1b, 1c, and 2 to 13. The vector construct shall comprise enough of the genomic sequence of the *glyt1* gene necessary for homologous recombination as well as part of the genomic sequence comprising part of an exon of the *glyt1* gene, or one exon or more exons of the *glyt1* gene framed by recognition sites of a recombinase. The vector construct may comprise all exons of the *glyt1* gene, preferably exons 1c to 13.

The genomic sequence in the vector construct comprises a positive selection marker, wherein the positive selection marker may be selected from the group comprising a neomycin resistance gene and a hygromycin resistance gene. The positive selection marker may also be framed by recognition sites for a recombinase, which allows for excision of the positive selection marker gene after selection of successful homologous recombination events. Thereby, any effect of the expression of the positive selection marker on the expression of the *glyt1* gene may be avoided. The recognition sites for a recombinase may be selected from the group comprising frt sites for a flp recombinase and lox sites for a cre recombinase. Preferably, the positive selection marker is a neomycin resistance gene. Optionally, the genomic sequence within the vector construct additionally comprises a negative selection marker, wherein the negative selection marker may be selected from the group comprising a diphtheria toxin gene and an HSV-thymidine kinase gene. Preferably, the negative selection marker is a diphtheria toxin gene.

In the genomic sequence of the *glyt1* gene at least part of a exon of *glyt1* gene is framed by recognition sites for a recombinase. This at least part of a exon of the *glyt1* gene may then be deleted with the help of a recombinase. The recognition sites for a recombinase may be selected from the group comprising frt sites for a flp recombinase and lox sites for a cre recombinase. Therefore, the part of the exon, or one exon or more exons of the *glyt1* gene, which encode part of the amino acid sequence of the GLYT1 protein necessary for its function and activity should be framed by recognition sites for a recombinase. In a preferred embodiment, exons 5 to 11 of the *glyt1* gene are framed by recognition sites for a recombinase.

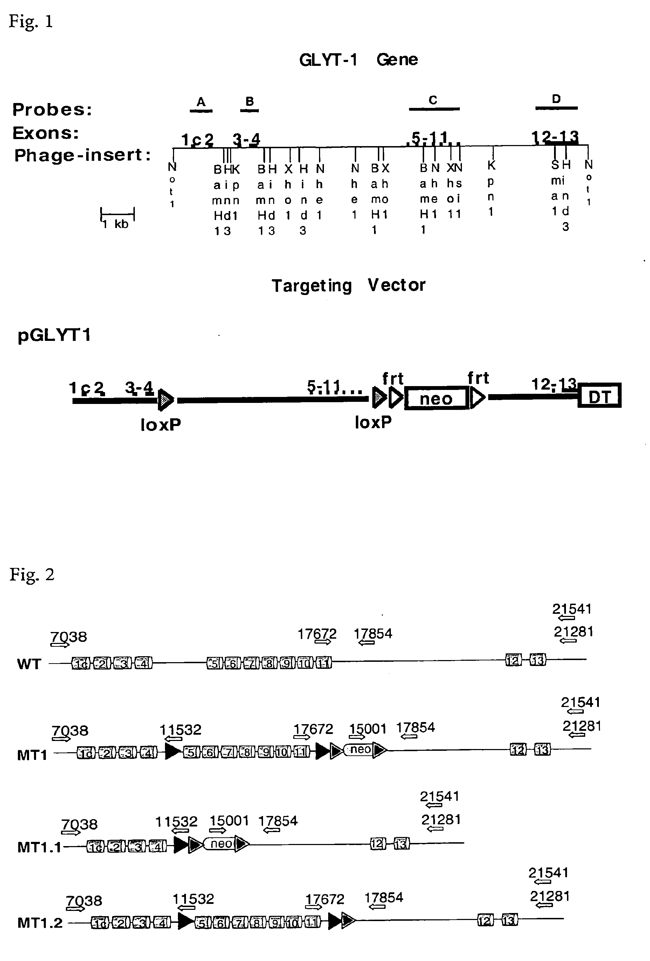

In a further embodiment, the pGLYT1 vector construct as depicted in Fig. 1 is provided.

In a further preferred embodiment, the genomic sequence of the *glyt1* gene is a murine sequence.

In another preferred embodiment, the vector construct comprises the nucleotide sequence of SEQ ID NO: 1.

The present invention further provides a method of producing a transgenic non-human animal, whose one or both alleles of a *glyt1* gene comprise a conditional targeted mutation comprising
(a) introducing a vector construct as described above into an embryonic stem cell,
(b) generating a heterozygous and/or homozygous transgenic animal from the said embryonic stem cell, and thereby
(c) producing a transgenic non-human animal, whose one or both alleles of a *glyt1* gene comprise a conditional targeted mutation.

In step (c) of the described method, a transgenic animal may be produced whose one or both alleles of a *glyt1* gene comprise the MT1 construct as depicted in Fig. 2.

The term transgenic animal as used herein comprises animals comprising a conditional targeted mutation in a gene, knock-out animals comprising a targeted null mutation in a gene, knock-in animals comprising a targeted insertion of a gene, and animals comprising a random insertion of a transgene. Animals comprising a conditional targeted mutation in a certain gene, as used herein, are animals wherein the said gene is modified in a way, that its protein-encoding function is not impaired and that upon recombination by a recombinase a functional mutation in the said gene may be generated.

In a further embodiment, the above-described method additionally comprises (d) further crossbreeding the transgenic animal produced in step (c) with an animal transgenic for the recombinase recognizing the recognition sites framing the selection marker within the genomic sequence of the *glyt1* gene.

In step (d) of the described method, a transgenic animal may be produced whose one or both alleles of a *glyt1* gene comprise the MT1.2 construct as depicted in Fig. 2.

In another embodiment, a method of producing a transgenic non-human animal is provided, whose one or both alleles of a *glyt1* gene are mutated and/or truncated in a way that less or no active GLYT1 protein is expressed comprising
(e) crossbreeding the transgenic animals produced in the above-described method in step (c) or the transgenic animal produced in step (d) with an animal transgenic for the recombinase recognizing the recognition sites framing the at least part of a exon of the *glyt1* gene, and thereby
(f) producing a transgenic non-human animal, whose one or both alleles of a *glyt1* gene are mutated and/or truncated in a way that less or no active GLYT1 protein is expressed.

In step (f) of the described method, a transgenic animal may be produced whose one or both alleles of a *glyt1* gene comprise the MT1.1 construct as depicted in Fig. 2.

Based on the specific conditional targeting construct tissue-specific or inducible GLYT1-deficient animals may be generated. These animals may be analyzed genetically, histologically, electrophysiologically, and behaviorally.

In a further embodiment, in the animal transgenic for the recombinase recognizing the recognition sites framing the at least part of a exon of the *glyt1* gene, said recombinase is expressed under the control of a tissue-specific promoter system. A tissue-specific promoter system may be any promoter system, which controls and directs expression of a gene in a tissue-specific manner, e.g., in brain tissue, in muscle tissue, in liver tissue, in kidney tissue etc. Examples of promoters known to induce specific expression in cells present in the brain comprise metallothionein III promoter, neuron specific enolase (NSE) promoter, sodium channel promoter, neurofilament M (NF-M) promoter, neurofilament L (NF-L) promoter, neurofilament H (NF-H) promoter, glial fibrillary acidic protein (GFAP) promoter, myelin basic protein (MBP) promoter, Proteolipid protein (Plp) promoter, Tyrosine Hydroxylase (TH) promoter, aldolase C promoter, synapsin I promoter, rhombotin 1 promoter, dopamine beta hydroxylase (DBH) promoter, choline acetyltransferase (ChAT), and Purkinje cell (Pcp2) promoter.

In even another embodiment, the recombinase in said animal transgenic for the recombinase is expressed under the control of a controllable promoter system. A controllable promoter system may be any promoter system, which controls the expression of a transgene in a regulatable and/or inducible fashion, e.g., by addition of specific inducer or repressor substances. Several inducible bacterial promoter systems are known in the art (Schultze N, Burki Y, Lang Y, Certa U, Bluethmann H; Nat Biotechnol 1996; 14(4): 499-503; van der Neut R; Targeted gene disruption: applications in neurobiology; J Neurosci Methods 1997; 71(1): 19-27; Liu HS, Lee CH, Lee CF, Su IJ, Chang TY; Lac/Tet dual-inducible system functions in mammalian cell lines. Biotechniques. 1998; 24(4): 624-8, 630-2).

In a further embodiment, a method of producing a transgenic non-human animal is provided, whose one or both alleles of a *glyt1* gene are mutated and/or truncated in a way that less or no active GLYT1 protein is expressed comprising
(a) introducing a vector construct as described into an embryonic stem cell,
(b) further introducing into said embryonic stem cell a vector construct comprising a nucleotide sequence encoding the recombinase recognizing the recognition sites framing the at least part of a exon of the *glyt1* gene,
(c) generating a heterozygous and/or homozygous transgenic animal from the said embryonic stem cell, and thereby
(d) producing a transgenic non-human animal, whose one or both alleles of a *glyt1* gene are mutated and/or truncated in a way that less or no active GLYT1 protein is expressed.

In step (d) of the described method, a transgenic animal may be produced whose one or both alleles of a *glyt1* gene comprise the MT1.1 construct as depicted in Fig. 2.

The above-described method may further comprise following step (b) step
(c) further introducing into said embryonic stem cell a vector construct comprising a nucleotide sequence encoding the recombinase recognizing the recognition sites framing the selection marker within the genomic sequence of the *glyt1* gene,
(d) generating a heterozygous and/or homozygous transgenic animal from the said embryonic stem cell, and thereby
(e) producing a transgenic non-human animal, whose one or both alleles of a *glyt1* gene are mutated and/or truncated in a way that less or no active GLYT1 protein is expressed.

Transgenic animals comprising targeted mutations are achieved routinely in the art as provided by Capecchi (Capecchi, M.R., Trends Genet, 1989. 5(3): p. 70-6) and Thomas et al. (Thomas, K.R. and M.R. Capecchi, Cell, 1987. 51(3): p. 503-12).

For example, the heterozygous and/or homozygous transgenic animal of the above-described methods may be generated by selecting recombinant embryonic stem cell clones, verifying the targeted mutation in the recombinant embryonic stem cell clones, injecting the verified recombinant embryonic stem cells into blastocysts, breeding chimeras from the blastocysts, investigating the chimeras for germ line transmission of the targeted mutation, breeding heterozygous animals, and optionally crossbreeding those heterozygous transgenic animals to generate homozygous transgenic animals.

Cell culture based models can also be prepared by two methods. Cell cultures can be isolated from the transgenic animals or prepared from established cell cultures using the same constructs with standard cell transfection techniques.

Embryonic stem cells used in the art which may also be used in the methods of this invention comprise C57BL/6J embryonic stem cells, BALB/c embryonic stem cells, and DBA/2J embryonic stem cells, CBA/J embryonic stem cells and embryonic stem cell lines of mouse strains 129.

The present invention further provides the transgenic non-human animal produced by any of the above described the methods.

In another embodiment of the invention, a transgenic non-human animal whose one or both alleles of a *glyt1* gene comprise a conditional targeted mutation are provided. In a preferred embodiment, the conditional targeted mutation comprises recognition sites for a recombinase framing part of the *glyt1* genomic sequence encoding amino acids essential for GLYT1 activity.

In a further embodiment, a transgenic non-human animal is provided whose one or both alleles of a *glyt1* gene comprise the construct MT1 or the construct MT1.2 as depicted in Fig. 2.

Based on the specific conditional targeting construct tissue-specific or inducible GLYT1-deficient animals may be generated. These animals may be analyzed genetically, histologically, electrophysiologically, and behaviorally.

The invention further relates to a transgenic non-human animal whose one or both alleles of a *glyt1* gene are mutated and/or truncated in a way that less or no active GLYT1 protein is expressed. In a preferred embodiment, the transgenic non-human animal comprises in one or both alleles a *glyt1* gene, wherein exons 5 to 11 are deleted. In a further embodiment, a transgenic non-human animal is provided whose one or both alleles of a *glyt1* gene comprise the construct MT1.1 as depicted in Fig. 2.

In another preferred embodiment, the transgenic non-human animal comprises in one or both alleles instead of the native *glyt1* gene the nucleotide sequence of SEQ ID NO: 1.

In the described transgenic animals whose one or both alleles of a *glyt1* gene are mutated and/or truncated in a way that less or no active GLYT1 protein is expressed, a modulation of NMDA receptor activity is expected *in vivo* by the alteration of the endogenous glycine level. Due to a lack of GLYT2 receptors in hippocampal and cerebral cortex regions a disruption of GLYT1 in these regions is expected to lead to elevated levels of glycine in glutamatergic synapses, thus enhancing NMDA-receptor function. In these animals an alteration of cognitive functions may occur, as well as a possible altered susceptibility to the induction of a psychotic-like symptomatology.

The transgenic non-human animal may be any animal known in the art, which may be used for the methods of the invention. Preferably, the animals of the invention are mammals, more preferred as a transgenic animal of the invention is a rodent. The most preferred transgenic animal is a mouse.

The present invention also relates to descendants of the transgenic non-human animals as provided by the invention, obtained by breeding with the same or with another genotype.

The present invention further provides a cell line or primary cell culture, tissue as well as an organotypic brain slice culture derived from the transgenic non-human animals as provided by the invention or its descendants.

Integration of the transgene comprising the conditional targeted mutation can be detected by various methods comprising genomic Southern blot and PCR analysis using DNA isolated from tail biopsies of two to three weeks old mice.

It will be apparent to the person skilled in the art that there are a large number of analytical procedures which may be used to detect the expression of the transgene comprising methods at the RNA level comprising mRNA quantification by reverse transcriptase polymerase chain reaction (RT-PCR) or by Northern blot, *in situ* hybridization, as well as methods at the protein level comprising histochemistry, immunoblot analysis and *in vitro* binding studies. Quantification of the expression levels of the targeted gene can moreover be determined by the ELISA technology, which is common to those knowledgeable in the art.

Quantitative measurement can be accomplished using many standard assays. For example, transcript levels can be measured using RT-PCR and hybridization methods including RNase protection, Northern blot analysis, and RNA dot analysis. APP and A-beta levels can be assayed by ELISA, Western blot analysis, and by comparison of immunohistochemically stained tissue sections. Immunohistochemical staining as well as immuno-electron microscopy can also be used to assess the presence or absence of the GLYT1 protein. Specific examples of such assays are provided below.

"Polynucleotide" and "nucleic acid" refer to single or double-stranded molecules which may be DNA, comprised of the nucleotide bases A, T, C and G, or RNA, comprised of the bases A, U (substitutes for T), C, and G. The polynucleotide may represent a coding strand or its complement. Polynucleotide molecules may be identical in sequence to the sequence, which is naturally occurring or may include alternative codons, which encode the same amino acid as that which is found in the naturally occurring sequence (see, Lewin "Genes V" Oxford University Press Chapter 7, 1994, 171-174). Furthermore, polynucleotide molecules may include codons, which represent conservative substitutions of amino acids as described. The polynucleotide may represent genomic DNA or cDNA.

The transgenic animals of the invention may be further characterized by methods known in the art, comprising immunohistochemistry, electron microscopy, Magnetic Resonance Imaging (MRI) and by behavioral studies addressing neurological and cognitive functions. Examples of behavioral tests are: spontaneous behavior, behavior related to cognitive functions, pharmacologically-disrupted behavior, grip strength, wire manoeuvre, swim test, rotarod, locomotor activity, Morris water maze, Y-maze, light-dark preference, passive and active avoidance tests.

A further objective of the present invention is the use of the transgenic non-human animal as described, or a cell line or tissue or an organotypic brain slice culture as derived thereof, as a model for studying the susceptibility to compounds inducing psychotic behavior. Additionally, these transgenic animals, cells or tissue or organotypic brain slice culture as derived thereof, may be used as a model for studying the effects of enhancing synaptic NMDA receptor function. Furthermore, these transgenic non-human animal, or cells or tissue or organotypic brain slice cultures derived thereof, may be used as a tool for assessing GLYT1 function.

In a further embodiment, a method for evaluating the *in vivo* effects of GLYT1 function on NMDA receptor activation is provided, comprising determining NMDA receptor activity, synaptic plasticity and behavior comprising learning and memory in a transgenic non-human animal, whose one or both alleles of a *glyt1* gene are mutated and/or truncated in a way that less or no active GLYT1 protein is expressed, and comparing the NMDA receptor activity, synaptic plasticity and behavior to those in an animal comprising a native *glyt1* gene.

In another embodiment, a method of testing GLYT1 inhibitor compounds for effects other than GLYT1-specific effects is provided, which method comprises administering a GLYT1 inhibitor compound to a transgenic non-human animal whose one or both alleles of a *glyt1* gene are mutated and/or truncated in a way that less or no active GLYT 1 protein is expressed, or a cell line or primary cell culture or an organotypic brain slice culture derived thereof, and determining the effect of the compound comprising assessing behavior, electrophysiology and histology, and comparing the behavior, electrophysiology and histology to those of a control.

GLYT 1 inhibitor compounds which may be used in the method of the invention are any GLYT 1 inhibitor compounds known in the art comprising ALX5407 (NPS Pharmaceuticals/NPS Allelix) and ORG24598 (Akzo/Organon).

Control may comprise any animal, cell line or primary cell culture or organotypic brain slice culture or tissue, wherein the *glyt1* gene is not mutated in a way, that less or no active GLYT 1 protein is expressed, or wherein the animal, cell line or primary cell culture or organotypic brain slice culture comprises the native *glyt1* gene. Assessment of the behavior may comprise spontaneous behavior, behavior related to cognitive functions comprising spatial short- and long-term memory, object recognition memory, associative emotional memory, conditioned fear extinction, and pharmacologically-disrupted behavior comprising drug-induced hyperlocomotion, drug-induced social withdrawal, drug-induced deficits in prepulse inhibition and drug-induced memory loss.

The present invention further relates to a kit for testing GLYT1 inhibitor compounds for effects other than GLYT1-specific effects comprising a transgenic non-human animal whose one or both alleles of a *glyt1* gene are mutated and/or truncated in a way that less or no active GLYT1 protein is expressed, or a cell line or primary cell culture or tissue or an organotypic brain slice culture or tissue derived thereof, and a means for determining whether a GLYT1 inhibitor exhibits effects other than GLYT1-specific effects.

Furthermore, the use of a transgenic non-human animal, whose one or both alleles of a *glyt1* gene are mutated and/or truncated in a way that less or no active GLYT1 protein is expressed, or a cell line or primary cell culture or tissue or an organotypic brain slice culture or tissue derived thereof is provided for testing of GLYT1 inhibitor compounds for effects other than GLYT1-specific effects.

Effects other than GLYT1-specific effects maybe any side-effects of a GLYT1 inhibitor compound produced by its interaction with any other molecule.

The invention further provides the transgenic animals, methods, compositions, kits, and uses substantially as described herein before especially with reference to the foregoing Examples.

Having now generally described this invention, the same will become better understood by reference to the specific examples, which are included herein for purpose of illustration only and are not intended to be limiting unless otherwise specified, in connection with the following figures.

### Figures

**Figure 1:** Top: Structure and restriction map of the 15 kb genomic *glyt1* clone, which encompasses exons 1c to 13. The probes (A- D) used for library screening and exon sequences are shown as black bars. Restriction sites are given below the gene map. Bottom: Gene targeting construct pGLYT1. Exons are shown as black bars with numbers of exons, recombination sites as triangles, and the neomycin-resistance gene (neo) and the diphtheria toxin (DT) gene as boxes.
**Figure 2:** Schematic diagram of the primer pairs used for the verification of recombination events.

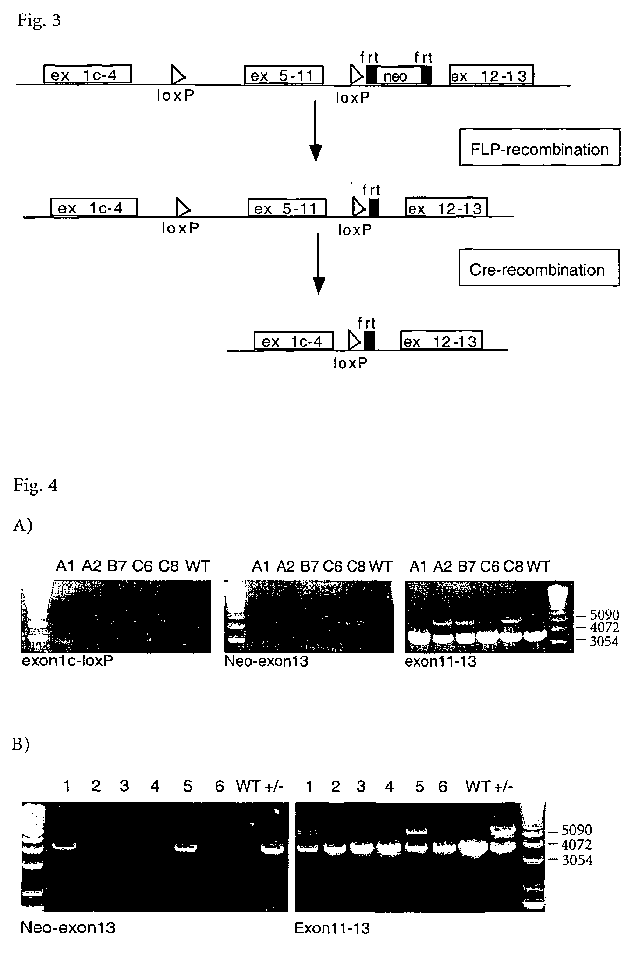

**Figure 3:** Schematic diagram for the site-directed recombination at the *glyt1* -locus.
**Figure 4:** A) left: PCR for mouse *glyt1* gene 5' of exon 1c to 5' loxP-site (SEQ ID NOs: 10 and 11). The amplicon resulting from the mutant allele has a size of 4494 bp, the wildtype allele gives no amplicon. A) middle and B) left: PCR for mouse *glyt1* gene neo-cassette to 3' of exon 13 (SEQ ID NOs: 15 and 14). The amplicon resulting from the mutant allele has a size of 4429 bp, the wildtype allele gives no amplicon. A) right and B) right: PCR for mouse *glyt1* gene exon 11 to 3' of exon 13 (SEQ ID NOs: 12 and 13 or 14). The amplicon resulting from the mutant allele has a size of 5351 (A) or 5611 (B) bp, the wildtype allele gives an amplicon of 3609 (A) or 3869 (B) bp.
   A1, A2, B7, C6, C8: ES-cell clones; WT: wildtype B6-DNA; 1 to 6: F1-mice; +/-: heterozygous control.

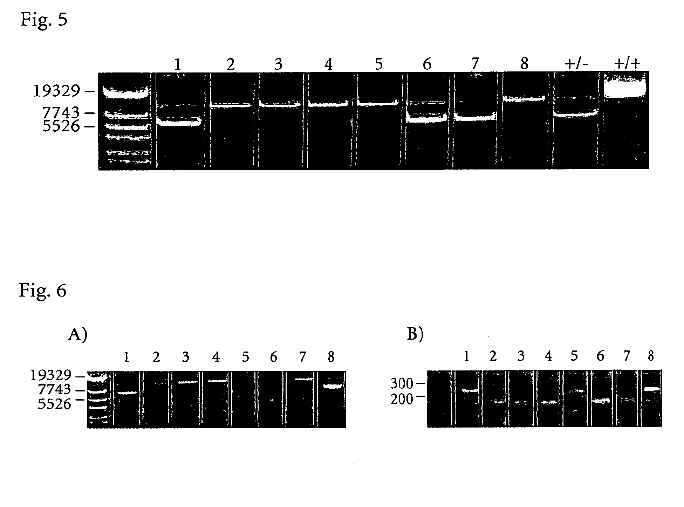

**Figure 5:** PCR for mouse *glyt1* gene 5' of exon 1c to 3' of exon 11 (SEQ ID NOs: 10 and 16) after Cre-recombination. The amplicon resulting from the cre-mutated allele has a size of 6238 bp, the wildtype allele gives an amplicon of 10816 bp. 1 to 8: F1-mice; +/-: heterozygous control; +/+: wildtype-control
**Figure 6:** Left: PCR for mouse *glyt1* gene 5' of exon 1c to 3' of exon 11 (SEQ ID NOs: 10 and 16) after Flp-recombination. The amplicon resulting from the Flp-mutated allele has a size of 7039 bp, the wildtype allele gives an amplicon of 10816 bp. Right: PCR for mouse *glyt1* gene exon 11 to 3' of exon 11 (SEQ ID NOs: 12 and 16). The amplicon resulting from the Flp-mutated allele has a size of 258 bp, the wildtype allele gives an amplicon of 182 bp. 1 to 8: F1-mice

### Examples

Commercially available reagents referred to in the examples were used according to manufacturer's instructions unless otherwise indicated.

### Example 1: Generation of transgenic mice

### A) Genomic cloning of glyt1

As a prerequisite for its targeted disruption the *glyt1* gene was cloned. Based on partial genomic sequences of mouse *glyt1* (Adams, R.H., et al., J Neurosci, 1995. 15(3 Pt 2): p. 2524-32) four genomic DNA segments encompassing exons 1c to 2, exons 3 to 4, exons 5 to 11, and exons 12 to 13 were amplified by PCR (primer with SEQ ID NOs: 2 and 3 for probe A; SEQ ID NOs: 4 and 5 for probe B; SEQ ID NOs: 6 and 7 for probe C; SEQ ID NOs: 8 and 9 for probe D). The amplified DNA segments were used as probes to screen a genomic library constructed from DNA of the ES cell line mJAX32 (129J/Ems). One 15 kb clone was isolated that hybridized to all four exon-probes. This clone was mapped and sequenced and found to contain the complete coding sequence of the *glyt1* gene (Fig. 1).

### B) Construction of glyt1 gene targeting vector

To target *glyt1* in ES cells a conditional gene-disruption vector was constructed to obtain mice with a tissue- or time-restricted deficiency of GLYT1.

The construct pGLYT1 encompasses a total of 10 kb of sequences with homology to the mouse *glyt1* gene (Fig. 1, SEQ ID NO: 1) cloned into the vector pBluescript. It is a replacement type gene targeting vector allowing both positive and negative selection. For positive selection a frt-flanked neomycin resistance gene has been inserted into intron 11 of the *glyt1* gene. To permit negative selection a diphtheria toxin gene has been added to the 3'-end of the vector. In order to achieve a conditional disruption of the *glyt1* gene loxP sites have been inserted flanking 5 kb of the *glyt1* gene including exons 5 to 11. In pGLYT1 mice the neomycin-resistance gene may be excised by Flp-mediated recombination at the frt sites in order to exclude any artifacts of gene interference (see E).

### C) Generation of B6-glyt1^{tml} mice

C57BL/6J embryonic stem cells (Eurogentec) were cultured and transfected with NotI-linearized targeting vector pGLYT1 (Capecchi, M.R., Trends Genet, 1989. 5(3): p. 70-6). Selected clones were screened for homologous recombination by PCR (Fig. 2). A correctly targeted clone (IC6, Fig.4A) carrying the mutation in the *glyt1* allele was used for injection into Balb/c host blastocysts. Chimeric males born after implantation of injected blastocysts into foster mothers were mated with C57BL/6J females, and offspring were analyzed for germline transmission of the *Glyt1*^{tml} mutation by PCR analysis (SEQ ID NOs: 10 to 15; Fig. 4B). Heterozygous B6-*Glyt1*^{tml +/-} mice were intercrossed to obtain homozygously mutated animals. Heterozygous animals are viable and do not show any macroscopic phenotype.

### D) Generation of B6-glyt1^{tml.1} mice

The same clone (IC6) used in C) was used for Cre-recombination to excise exons 5 to 11, flanked by loxP-sites in vitro. ES cells were propagated to 1.5 x 10⁷ cells and electroporated with 20 µg supercoiled pMC-Cre (Thomas, K.R. and M.R. Capecchi, Cell, 1987. 51(3): p. 503-12). Single colonies were picked and screened for site-specific recombination by PCR (SEQ ID NOs: 10 and 16). A correctly recombined clone (IIB2) carrying the deletion in the *glyt1* allele was used for injection into Balb/c host blastocysts. Chimeras were bred and tested as described under C) (Fig.5). Heterozygous animals are viable and do not show any macroscopic phenotype.

### E) Generation of B6-glyt1^{tml.2} mice

The same clone (IC6) used in C) was used for Flp recombination to excise the neomycin-resistance gene flanked by frt sites in vitro. ES cells were propagated to 1.5 x 10⁷ cells and electroporated with 20 µg supercoiled plasmid for expression of Flp recombinase (Gu, H., Y.R. Zou, and K. Rajewsky, Cell, 1993. 73(6): p. 1155-64). Single colonies were picked and screened for site-specific recombination by PCR (SEQ ID NOs: 10, 12 and 16). A correctly recombined clone (IIE5) was used for injection into Balb/c host blastocysts. Chimeras were bred and tested as described under C) (Fig.6). Heterozygous animals are viable and do not show any macroscopic phenotype.

### Example 2: Molecular analysis of GLYT1 mutant mice

### 1) Histological analysis of GLYT1 mutant mice

The expression of GLYT1 in the brains of mutant mice is analyzed immunohistochemically using the GLYT1-specific antibodies raised in rabbits and guinea pigs. GLYT1 expression in GLYT1-mutant mice and wild-type mice is correlated to the expression pattern of NMDA receptors.

### 2) Electrophysiological analysis of GLYT1 mutant mice

NMDA receptor activation is required for induction of certain forms of long term potentiation (LTP) (Bliss, T.V. and G.L. Collingridge, Nature, 1993. 361(6407): p. 31-9). Potentiation induced by theta burst stimulation in hippocampal slices of GLYT1-mutants is compared with wild-type controls throughout the post-tetanus period as described previously (Kew, J.N., et al., J Neurosci, 2000. 20(11): p. 4037-49). It is determined whether GLYT1-mutant mice exhibit a heightened sensitivity to the induction of LTP compared to controls.

### 3) Preparation of forebrain and brainstem synaptosomes

Mice are sacrified and brain tissue are dissected on ice and subsequent procedures performed at 4°C. Tissues are homogenized in 10 Vol (w/v) of 10 mM Tris-HCl pH 7.4 containing 0.32 M sucrose and 1 mM Pefabloc (cocktail of protease inhibitors) (buffer A) using a glass/teflon homogeniser (800 rpm 10 times). The homogenate is centrifuged 5' at 1300 x g. The supernatant is carefully decanted and kept on ice, while the pellet is suspended in 5 vol (of the original weight) of buffer A, homogenised and centrifuged as described. The second supernatant is added to the first and centrifuged at 17,000 x g for 20'. The resulting pellet (crude synaptosomal fraction) is suspended in 5 vol (of the original weight) of Krebs-Ringer solution, pH 7.4 containing 10 mM glucose (KRB).

### 4) Glycine uptake

The assays are performed in 96-well plates. Aliquots of mice forebrain (0.1mg) and brainstem (0.05 mg) synaptosomal preparations are incubated at 22°C in KRB together with 120 nM [3H] glycine in a total volume of 250 µl for 30'. Incubation is stopped by rapid filtration onto 96 well Packard GF/B unifilter plates, followed by 3 washes with ice-cold KRB. After addition of scintillation solution the radioactivity content of the wells is measured.

### 5) Saturation binding for MK801

The assays are performed in 96-well deep plates. Aliquots of mice forebrain and brainstem synaptosomal preparations (0.07 mg) are incubated at 22°C in 20 mM Hepes-KOH, pH 7.4, containing 100 µM glutamate and 30µM glycine together with increasing concentration (0.03nM- 300 nM) of [3H] MK801 in a total volume of 0.5 ml for 1 hour. Non-specific binding is defined with 10 µM MK801. Incubation is stopped by rapid filtration onto 96 well Packard GF/C unifilter plates, followed by 3 washes with ice-cold 20 mM of Hepes-KOH, pH 7.4. After addition of scintillation solution the radioactivity content of the wells is measured.

### Example 3: Behavioral analysis of GLYT1-deficient mice

An enhancement of NMDA-receptor function in the GLYT1-deficient mice may be expected to have an influence on cognitive functions and to alter the susceptibility to drugs comprising apomorphine, D-amphetamine, phencyclidine (PCP) which have effects on schizophrenia-type symptoms.

### 1) Spontaneous behaviour

The GLYT1-deficient mouse lines are observed for signs of natural exploratory behaviour including body posture, gait and sensory responses (Gossen, M. and H. Bujard, Proc Natl Acad Sci U S A, 1992. 89(12): p. 5547-51). In addition, their locomotor activity is analyzed (activity box) as well as their motor coordination (rotarod test). Moreover, the state of anxiety is assessed by exposing to the animals to naturally aversive stimuli (elevated plus maze test and the light/dark choice test).

### 2) Behaviour related to cognitive functions

### Spatial short- and long-term memory

The delayed matching spatial working memory task is performed as described by Durkin (Irwin, S., Psychopharmacologia, 1968. 13(3): p. 222-57). Working memory is evaluated on the basis of the acquisition of a delayed matching rule in a 5-arm maze. The basic learning task is comprised of two phases. Each trial begins with a presentation phase during which the animal is exposed to a forced and rewarded visit to one arm chosen quasi-randomly, the other four arms being closed. Once rewarded, the animal is placed in a waiting cage. Following a retention interval of variable duration (2-s, 20-s and 40-s delay for working memory; 5-min, 1-hr, 4-hr, 24-hr delay for short- and long-term memory), the retrieval test phase is performed during which the animal is exposed to a situation of choice among the five open arms. A correct choice of the previously visited arm is rewarded. The working memory retention capacity is expressed as a function of the retention interval by the mean percent of correct accuracy choices during successive trials with a fixed intertrial interval of 10 s. The memory task is monitored by an automated video-tracking system.

Alternatively, spatial learning and memory is assessed in the water maze paradigm described by Morris (Durkin, T.P., et al., Behav Brain Res, 2000. 116(1): p. 39-53 and Morris, R.G., et al., Nature, 1982. 297(5868): p. 681-3). Mice are placed in a circular pool (diameter 120 cm, height 30 cm) in which they learn to escape from milky water (20 cm depth, 20 ± 1°C) by locating a hidden platform. This target platform (7 cm diameter, 1 cm below the water surface) is located in the center of a particular quadrant of the pool, and external visual cues are positioned around the pool to facilitate navigation of the animals. During a 4 d test period, mice are placed in the water facing the wall of the pool in one of four fixed starting positions chosen randomly (3 trials per session, 3 sessions per day). The time the mouse needs to locate the target (escape latency) and the swim path and swim speed are measured using an automated video motility system. If an animal fails to find the target within 60 seconds, it is placed on the platform by hand and is allowed to remain there for an intertrial interval (10-20 sec). The interval between each session is 1.5 - 2 hr. After the final trial on day 4, the platform is removed, and the mice are allowed to swim freely for 60 sec. The time the mice spend in each quadrant and their swim path are recorded.

### Object recognition memory task

The visuospatial recognition memory task is an adaptation of the object recognition memory test initially developed by Ennaceur and Delacour (Morris, R.G., et al., Nature, 1986. 319(6056): p. 774-6). The evaluation of the memory retrieval is based on a delayed non-matching to sample rule. Mice are familiarized with the test apparatus, which consists of a T-shape oriented maze with a West, East and South arm spatially defined by specific extramaze cues. The visuospatial recognition memory session comprises two consecutive 5-min trials. For the first acquisition trial, mice are exposed to three different novel objects (O1, O2, and O3) that are positioned at a distinct location (e.g. O1/West, O2/East, and O3/South) in the T-shape oriented maze. The number and the duration of exploration are measured for each object during 5 minutes. Following a variable intertrial retention interval (1-hr, 4-hr and 24-hr interval), the recognition trial is performed. One of the three familiar objects (familiar target) is presented with two novel objects 04 and 05 within the same spatial arrangement (e.g. O1/West in the T-shape maze orientation) to assess visual retrieval-related memory. In addition, the objects are presented in a new spatial arrangement (e.g. O1/East in the inverted T-shape maze orientation) to assess spatial retrieval-related memory. The object and spatial recognition memory capacity is expressed as a function of the intertrial retention interval by the difference in the number and duration of exploration of the familiar target between the first and the second trial.

### Associative emotional memory

Associative emotional memory, which is NMDA receptor dependent, is assessed in two behavioral tasks:

Fear potentiated startle response: Mice are conditioned to respond to a light (CS) which is paired with a footshock (aversive US). After a delay of 24 hours, emotional memory is evaluated by the amplitude of the startle reflex elicited by different acoustic stimuli (90-110 dB) with or without the conditioned light stimulus. The presentation of the conditioned light stimulus leads to a potentiation of the acoustic startle reflex (Ennaceur, A. and J. Delacour, Behav Brain Res, 1988. 31(1): p. 47-59).

Contextual fear conditioning: Contextual fear conditioning is an implicit aversive associative learning process by which an initially neutral context acquires aversive properties after its repetitive association with an unconditioned aversive stimulus (US). The animals are exposed to a new chamber where they are treated after few minutes to successive electric foot shocks (US) that elicit unconditioned fear responses (freezing behavior) (Davis, M., Psychopharmacology (Berl), 1979. 62(1): p. 1-7). Contextual fear conditioning is measured by the amount of freezing in response to re-exposure to the context. The conditioned freezing response is tested at different periods of time after training in order to evaluate short-term (1-3 hrs) and long-term (1-10 days) contextual memory.

### Conditioned fear extinction

Extinction of a learned fear response represents a form of behavioral plasticity that is thought to rely on the formation of a new form of memory rather than an erasure of the original learned association (Phillips, R.G. and J.E. LeDoux, Behav Neurosci, 1992. 106(2): p. 274-85). Recently it has been shown that conditioned fear extinction involves an NMDA receptor-dependent process (Tang, Y.P., et al.,Nature, 1999. 401(6748): p. 63-9). Following a delay of 24-hr after training, extinction of conditioned freezing can be evaluated by the time-dependent decrease of the amount of freezing in response to repetitive exposure to the context during five consecutive days.

### 3) Pharmacologically-disrupted behavior

A distinct range of schizophrenia-type symptoms, including hyperlocomotion, deficits in prepulse inhibition (PPI) and memory deficit can be induced by the administration of apomorphine, D-amphetamine or the non-competitive NMDA receptor antagonist PCP. The minimally effective dose in disrupting behavior in wildtype mice is used. It is then tested whether GLYT1 mutant mice are resistant, less or more sensitive to the pharmacological disruption of behavior.

### Drug-induced hyperlocomotion

In the open field (activity box) the effect of D-amphetamine on locomotor activity and stereotypic behavior is assessed by recording the horizontal locomotor activity, vertical activity, stereotypic movements and the time spent in the center of the open field.

### Drug-induced social withdrawal

To assess the involvement of GLYT1 mutations in the social behavioral response to D-amphetamine or PCP a social exploration test is used (Falls, W.A., M.J. Miserendino, and M. Davis, J Neurosci, 1992. 12(3): p. 854-63). An individually housed male mouse is exposed for 5 min. to a juvenile female mouse. Social interactions including ano-genital and neck sniffing, heterogrooming and pursuits are recorded via a video tracking system before and after drug administration. The drug-induced social exploration deterioration is examined at different time intervals (30 min, 2 hours, 4 hours and 24 hours) after drug administration.

### Drug-induced deficits in prepulse inhibition

Disruption of prepulse inhibition (PPI) by apomorphine or PCP is a frequently used model of the sensorimotor gating deficits (Crestani, F., F. Seguy, and R. Dantzer, Brain Res, 1991. 542(2): p. 330-5; Kretschmer, B.D., et al., Eur J Pharmacol, 1997. 331(2-3): p. 109-16 and Bakshi, V.P. and M.A. Geyer, J Neurosci, 1998. 18(20): p. 8394-401) by which attention and sensorimotor gating can be evaluated. PPI is the attenuation of an acoustic or tactile startle response following the presentation of a non-startle-inducing prepulse stimulus. The animal's startle response following acoustic stimuli is assessed.

### Drug-induced memory loss

PCP- or apomorphine-induced impairment of memory functions is assessed in the delayed matching spatial memory task described above.

## Claims

1. A vector construct comprising the whole or part of the genomic sequence of the *glyt1* gene, wherein the genomic sequence comprises a positive selection marker and optionally a negative selection marker and wherein at least part of a exon of the *glyt1* gene is framed by recognition sites for a recombinase.

2. The vector construct according to claim 1, wherein the genomic sequence comprises all exons of the *glyt1* gene.

3. The vector construct according to any one of claims 1 or 2, wherein the genomic sequence comprises a neomycin resistance gene as a positive selection marker.

4. The vector construct according to any one of claims 1 or 2, wherein the genomic sequence optionally comprises a diphtheria toxin gene as a negative selection marker.

5. The vector construct according to any one of claims 1 or 2, wherein the genomic sequence comprises a neomycin resistance gene as a positive selection marker and a diphtheria toxin gene as a negative selection marker.

6. The vector construct according to any one of claims 1 to 5, wherein the positive selection marker is framed by recognition sites for a recombinase.

7. The vector construct according to any one of claims 1 to 6, wherein exons 5 to 11 of the *glyt1* gene are framed by recognition sites for a recombinase.

8. The vector construct according to any one of claims 1 to 7, wherein the genomic sequence of the *glyt1* gene is a murine sequence.

9. The vector construct according to claim 1 comprising the nucleotide sequence of SEQ ID NO: 1.

10. A method of producing a transgenic non-human animal, whose one or both alleles of a *glyt1* gene comprise a conditional targeted mutation comprising
(a) introducing a vector construct according to any one of claims 1 to 9 into an embryonic stem cell,
(b) generating a heterozygous and/or homozygous transgenic animal from the said embryonic stem cell, and thereby
(c) producing a transgenic non-human animal, whose one or both alleles of a *glyt1* gene comprise a conditional targeted mutation.

11. The method according to claim 10, additionally comprising
(d) further crossbreeding the transgenic animal produced in step (c) with an animal transgenic for the recombinase recognizing the recognition sites framing the selection marker within the genomic sequence of the *glyt1* gene.

12. A method of producing a transgenic non-human animal, whose one or both alleles of a *glyt1* gene are mutated and/or truncated in a way that less or no active GLYT1 protein is expressed comprising any steps of the methods according to any one of claims 10 or 11, and further comprising
(e) crossbreeding the said transgenic animal with an animal transgenic for the recombinase recognizing the recognition sites framing the at least part of a exon of the *glyt1* gene, and thereby
(f) producing a transgenic non-human animal, whose one or both alleles of a *glyt1* gene are mutated and/or truncated in a way that less or no active GLYT1 protein is expressed.

13. The method according to claim 12, wherein in the animal transgenic for the recombinase recognizing the recognition sites framing the at least part of a exon of the *glyt1* gene, said recombinase is expressed under the control of a tissue-specific promoter system.

14. The method according to claim 12, wherein in the animal transgenic for the recombinase recognizing the recognition sites framing the at least part of a exon of the *glyt1* gene, said recombinase is expressed under the control of a controllable promoter system.

15. A method of producing a transgenic non-human animal, whose one or both alleles of a *glyt1* gene are mutated and/or truncated in a way that less or no active GLYT1 protein is expressed comprising
(a) introducing a vector construct according to any one of claims 1 to 9 into an embryonic stem cell,
(b) further introducing into said embryonic stem cell a vector construct comprising a nucleotide sequence encoding the recombinase recognizing the recognition sites framing the at least part of a exon of the *glyt1* gene,
(c) generating a heterozygous and/or homozygous transgenic animal from the said embryonic stem cell, and thereby
(d) producing a transgenic non-human animal, whose one or both alleles of a *glyt1* gene are mutated and/or truncated in a way that less or no active GLYT1 protein is expressed.

16. A method of producing a transgenic non-human animal, whose one or both alleles of a *glyt1* gene are mutated and/or truncated in a way that less or no active GLYT1 protein is expressed comprising
(a) introducing a vector construct according to any one of claims 1 to 9 into an embryonic stem cell,
(b) further introducing into said embryonic stem cell a vector construct comprising a nucleotide sequence encoding the recombinase recognizing the recognition sites framing the at least part of a exon of the *glyt1* gene,
(c) further introducing into said embryonic stem cell a vector construct comprising a nucleotide sequence encoding the recombinase recognizing the recognition sites framing the selection marker within the genomic sequence of the *glyt1* gene,
(d) generating a heterozygous and/or homozygous transgenic animal from the said embryonic stem cell, and thereby
(e) producing a transgenic non-human animal, whose one or both alleles of a *glyt1* gene are mutated and/or truncated in a way that less or no active GLYT1 protein is expressed.

17. A transgenic non-human animal produced by the method according to any one of claims 10 or 11.

18. A transgenic non-human animal whose one or both alleles of a *glyt1* gene comprise a conditional targeted mutation.

19. The transgenic non-human animal according to claim 18, wherein the conditional targeted mutation comprises recognition sites for a recombinase framing part of the *glyt1* genomic sequence encoding amino acids essential for GLYT1 activity.

20. The transgenic non-human animal according to any one of claims 17 to 19, wherein the animal is a rodent.

21. The transgenic non-human animal according to claim 20, wherein the rodent is a mouse.

22. A transgenic non-human animal produced by the method according to any one of claims 12 to 16.

23. A transgenic non-human animal whose one or both alleles of a *glyt1* gene are mutated and/or truncated in a way that less or no active GLYT1 protein is expressed.

24. The transgenic non-human animal according to claim 23, whose genome comprises in one or both alleles a *glyt1* gene, wherein exons 5 to 11 are deleted.

25. The transgenic non-human animal according to claim 24, whose genome comprises in one or both alleles instead of the native *glyt1* gene the nucleotide sequence of SEQ ID NO: 1.

26. The transgenic non-human animal according to any one of claims 22 to 25, wherein the animal is a rodent.

27. The transgenic non-human animal according to claim 26, wherein the rodent is a mouse.

28. Descendant of the transgenic non-human animal according to any one of claims 17 to 27, obtained by breeding with animals of the same or another genotype.

29. A cell line or primary cell culture derived from a transgenic non-human animal or its descendants according to any one of claims 22 to 28.

30. A tissue or an organotypic brain slice culture derived from a transgenic non-human animal or its descendants according to claims 22 to 28.

31. Use of a transgenic non-human animal according to claims 22 to 27 or a cell line or primary cell culture according to claim 29 or a tissue or an organotypic brain slice culture according to claim 30 as a model for studying the susceptibility to compounds inducing psychotic behavior.

32. Use of a transgenic non-human animal according to claims 22 to 27 or a cell line or primary cell culture according to claim 29 or a tissue or an organotypic brain slice culture according to claim 30 as a model for studying the effects of enhancing synaptic NMDA receptor function.

33. Use of a transgenic non-human animal according to claims 22 to 27 or a cell line or primary cell culture according to claim 29 or a tissue or an organotypic brain slice culture according to claim 30 as a tool for assessing GLYT1 function.

34. A method for evaluating the *in vivo* effects of GLYT1 function on NMDA receptor activation comprising determining NMDA receptor activity, synaptic plasticity and behavior comprising learning and memory in a transgenic non-human animal according to any one of claims 22 to 27, and comparing the NMDA receptor activity, synaptic plasticity and behavior to those in an animal comprising a native *glyt1* gene.

35. A method of testing GLYT1 inhibitor compounds for effects other than GLYT1-specific effects which method comprises administering a GLYT1 inhibitor compound to a transgenic non-human animal according to claims 22 to 27 or a cell line or primary cell culture according to claim 29 or a tissue or an organotypic brain slice culture according to claim 30, and determining the effect of the compound comprising assessing behavior, electrophysiology and histology, and comparing the behavior, electrophysiology and histology to those of a control.

36. A kit for testing GLYT1 inhibitor compounds for effects other than GLYT1-specific effects comprising a transgenic non-human animal according to any one of claims 22 to 27 or a cell line or primary cell culture according to claim 29 or a tissue or an organotypic brain slice culture according to claim 30, and a means for determining whether a GLYT1 inhibitor exhibits effects other than GLYT1-specific effects.

37. Use of a transgenic non-human animal according to any one of claims 22 to 27 or a cell line or primary cell culture according to claim 29 or a tissue or an organotypic brain slice culture according to claim 30 for testing of GLYT1 inhibitor compounds for effects other than GLYT1-specific effects.

38. The vector constructs, methods, transgenic animals, kits and uses substantially as described herein before especially with reference to the foregoing Examples.
